# EUROPÄISCHE PATENTSCHRIFT

(11) **EP 2 944 288 B1**
(45) Veröffentlichungstag und Bekanntmachung des Hinweises auf die Patenterteilung: **08.04.2020**
(21) Anmeldenummer: 14167896.1
(22) Anmeldetag: 12.05.2014
(51) Int. Cl.: A61C 13/15

(54) **Lichthärtgerät, insbesondere dentales Lichthärtgerät**
Light curing device, in particular dental light curing device
Appareil de durcissement à la lumière, en particulier dentaire

(43) Veröffentlichungstag der Anmeldung: 18.11.2015
(73) Patentinhaber: Ivoclar Vivadent AG, 9494 Schaan (LI)
(72) Erfinder: Pauler, Markus, 6800 Feldkirch (AT); Kögel, Alexander, 6800 Feldkirch (AT)
(74) Vertreter: Baronetzky, Klaus

(56) Entgegenhaltungen:
- EP-A1- 1 236 444
- EP-A1- 2 140 832
- WO-A1-94/26203
- WO-A1-2014/043488
- WO-A1-2014/135589
- US-A1- 2013 268 033

## Beschreibung

Die Erfindung betrifft ein Lichthärtgerät, insbesondere ein dentales Lichthärtgerät für zu polymerisierendes Material wie PMMA oder Komposit und Adhäsive, Zemente sowie für die Anregung beliebiger Photoinitiatoren, gemäß dem Oberbegriff von Anspruch 1.

Derartige Lichthärtgeräte sind seit langem bekannt und werden im großen Umfang eingesetzt, wenn es gilt, Komposits oder PMMA-Dentalrestaurationen zu polymerisieren und auszuhärten.

Für die Polymerisation der Dentalrestaurationen ist es erforderlich, diesen eine vorgegebene Lichtmenge zuzuleiten, was impliziert, dass nicht die Abgabe der Lichtmenge durch das Lichthärtgerät, sondern die Zuleitung der Lichtmenge zur Dentalrestauration entscheidend ist.

Seit langem ist es bekannt, hierzu sogenannte Lichtdosierungssensoren zu verwenden, wozu beispielhaft auf die DE 92 04 621 U1 zu verweisen ist. Durch die dortige Dosiereinrichtung soll das reflektierte Licht erfasst werden, und hiervon abhängig soll die Lichtmenge bereitgestellt werden, die erforderlich ist, um die vollständige Polymerisation des Dentalrestaurationsmaterials zu ermöglichen.

Wenn das Lichtauslassende des als Handgerät ausgebildeten Lichthärtgeräts in den Raum der Mundhöhle - aber nicht auf die Zähne - gerichtet ist, wird nahezu kein Licht reflektiert, so dass die dortige Dosiereinrichtung dieses Fehllicht bei der Bemessung der abzugebenen Lichtmenge berücksichtigt.

Wenn andererseits das Lichtauslassende auf einen Zahn gerichtet ist, jedoch neben das Restaurationsmaterial, erfolgt eine Reflektion des abgegebenen Lichts zur Dosiereinrichtung hin. Dieses reflektierte Licht wird von der Dosiereinrichtung nicht als Fehllicht berücksichtigt, und die Zeit, in der von der Lichtquelle des Lichthärtgeräts Licht abgegeben wird, wird bei der Berechnung des Polymerisationszyklus, also der gesamten Belichtungszeit des Dentalrestaurationsteils, berücksichtigt.

Dies führt zu einer Fehlkalkulation, denn tatsächlich wird durch die Beaufschlagung von benachbarten Zähnen - oder beispielsweise benachbarten, bereits ausgehärteten Dentalrestaurationen -, keine bzw. unzureichende Polymerisation vorgenommen.

Zwar soll durch ein Polarisationsfilter, der aber verzichtbar sein soll, eine Unterscheidung von Arten des reflektierten Lichts realisiert werden. Dies ist aber mindestens dann nicht möglich, wenn das Licht von einer benachbarten, ausgehärteten Dentalrestauration reflektiert wird.

Diese Lösung hat sich daher - insofern nicht ganz überraschend - nicht durchgesetzt.

Auch bei stationären Lichthärtgeräten sind Sensoren bekannt geworden, die der Steuerung des Lichthärtgeräts dienen. Beispielsweise sei hier auf die DE 32 25 589 A1 und die DE 82 19 588 U1 verwiesen. Über optische Sensoren wird bei diesen Lösungen ein stationäres Lichthärtgerät eingeschaltet, wenn das zu behandelnde Dentalrestaurationsteil sich dem Lichthärtgerät annähert.

Auch bei diesen Lösungen ist jedoch eine Fehlbelichtung möglich, beispielsweise, wenn der Finger des Benutzers sich dem optischen Näherungssensor nähert, bevor das Dentalrestaurationsteil in den Lichtkegel gebracht wird.

Die EP 2 140 832 beschreibt ein Lichthärtgerät, bei dem die von dem zu bestrahlenden Dentalobjekt reflektierte Strahlung erfasst und damit eine Überprüfung, ob das Lichthärtgerät korrekt über der zu bestrahlenden Fläche ausgerichtet ist, ermöglicht werden soll.

Auch die PCT/US2013/059686 beschreibt ein dentales Lichthärtgerät, bei dem neben der eigentlichen Strahlenquelle (blaues Licht), die der Aushärtung der photopolymerisierenden Kunststoffe dient, eine weitere Lichtquelle, die mit einer Bilderfassungsvorrichtung zusammenwirken und die für eine Bilderfassung nötige Beleuchtung des zu erfassenden Objektes zur Verfügung stellen soll. Über eine Darstellung des so erfassten Bildes soll eine vereinfachte manuelle Korrektur der Ausrichtung des Lichthärtgerätes möglich sein.

Die EP 1 236 444 beschreibt darüber hinaus ein Lichthärtgerät, bei dem die Lichtquelle mit geringer Leistung betrieben als so genanntes "Pilotlicht" fungieren können soll, wobei die vom zu bestrahlenden Objekt reflektierte Strahlung im Lichthärtgerät erfasst und bei Überschreiten eines Schwellwertes, der einen ausreichend geringen Abstand des Lichthärtgerätes vom zu bestrahlenden Objekt repräsentieren soll, eine Erhöhung der Lichtleistung der Lichtquelle auf volle Leistung (mit entsprechend hohem Blauanteil) bewirken soll. Somit soll gewissermaßen eine Einschaltung des Lichthärtgerätes bei zu großem Abstand zum zu bestrahlenden Objekt vermieden werden.

Die bislang bekannten Lichthärtgeräte erzeugen bei einer Fehlbedienung regelmäßig Fehlhärtungen, was umso kritischer ist, da heutzutage der Verbleib von Monomeren mit ihren freien Radikalen im Mund des Patienten häufig als Haftungsfall für die Zahnarzt angesehen wird.

Demgegenüber liegt der Erfindung die Aufgabe zu Grunde, ein Lichthärtgerät gemäß dem Oberbegriff von Anspruch 1 zu schaffen, das neben der verbesserten Sicherheit der Bedienung auch eine verbesserte Ergonomie bietet.

Diese Aufgabe wird erfindungsgemäß durch Anspruch 1 gelöst. Vorteilhafte Weiterbildungen ergeben sich aus den Unteransprüchen.

Durch die Realisierung eines Ortsensors und/oder eines Bewegungssensors und/oder eines Winkelsensors und/oder eines Magnetfeldsensors und/oder deren Kombination als Teil des Lichthärtgeräts lassen sich gleich mehrere Vorteile erzielen. Der eingesetzte Sensor kann beispielsweise verwendet werden, um sicherzustellen, dass das Lichthärtgerät an der Stelle verbleibt, an der der Zahnarzt es beim Einschalten zur Polymerisation eingesetzt hat.

Damit soll sichergestellt werden, dass - wenn überhaupt eine Polymerisation vorgenommen wird - diese vollständig vorgenommen wird. Der Zahnarzt erkennt demgegenüber ohne weiteres das unpolymerisierte Dentalrestaurationsteil, da es weich bzw. nahezu flüssig ist. In diesem Zusammenhang sei darauf hingewiesen, dass der Zahnarzt nicht ohne weiteres erkennen kann, wenn die Oberfläche des Inkrements aushärtet, in der Tiefe/auf der Unterseite der Füllung aber keine Aushärtung stattfindet.

Gemäß einem vorteilhaften Aspekt der Erfindung geht diese von dem Gedanken aus, dass, wenn der Zahnarzt das Lichthärtgerät an der richtigen Stelle ansetzt und die Belichtung auslöst, also den Polymerisationszyklus startet, dieser auch ordnungsgemäß vorgenommen wird, wenn er das Gerät nicht versehentlich weg bewegt.

Dies lässt sich mit den erfindungsgemäßen Ortsensoren bzw. Bewegungssensoren bzw. Magnetfeldsensoren bzw. Gyrosensoren bzw. deren Kombination sicherstellen, die erfassen, wenn das Lichthärtgerät nach dem Start der Belichtung weg bewegt wird.

Hierzu sind die erfindungsgemäßen Sensoren bevorzugt als Relativsensoren und/oder Absolutsensoren ausgebildet. Ein derartiger Ortsensor erkennt eine Bewegung des Handgeräts, als das das Lichthärtgerät ausgebildet ist, in einer der drei Raumrichtungen oder Dimensionen, bevorzugt aber auch eine Winkeländerung.

In diesem Zusammenhang ist es erfindungsgemäß bevorzugt, Grenzwerte vorzusehen, ab welchen eine Bewegung als kritisch angesehen wird. Beispielsweise kann eine Winkelabweichung der Ausrichtung des Lichtstrahls, der von dem Lichtauslassende abgegeben wird, deutlich kritischer sein als eine Verlagerung des Lichthärtgeräts in axialer Richtung, also in Richtung des Lichtabgabeelements, um einen oder mehrere Milimeter. Die Winkelabweichung würde dazu führen, dass das Zielgebiet des Lichtabgabelements, also die Dentalrestauration oder die Bearbeitungsoberfläche, deutlich verfehlt wird, während eine winkeltreue Verlagerung des Handgeräts in rückwärtiger Richtung lediglich zur - in der Regel geringen - Reduktion der aufgebrachten Lichtleistung führt.

Darüber hinaus ist es auch möglich, die durch den erfindungsgemäßen Ortsensor und/oder Bewegungssensor erfassten Parameter, die als Signale der erfindungsgemäßen Steuereinrichtung zugeleitet werden, auszuwerten, um eine Aktion oder Funktion des Lichthärtgeräts auszulösen.

Beispielsweise kann das Aufnehmen eines auf einer Unterlage abgelegten Handgeräts, als das das Lichthärtgerät ausgebildet ist, dazu erfasst werden, und die Erfassung dazu genutzt werden, das Lichthärtgerät in einen aktiven Zustand zu schalten, in dem unmittelbar der Polymerisationszyklus bei Bedarf gestartet werden kann.

Bei dieser Ausgestaltung ist das Lichthärtgerät bevorzugt in abgelegtem Zustand in einem Ruhezustand, der die für den Betrieb verwendeten Akkumulatoren schont. Die Aufnahme des Lichthärtgeräts ist mit einer Bewegung verbunden, die von den erfindungsgemäßen Sensoren erfasst wird.

Auch ist es möglich, durch gezielte Bewegungen, wie beispielsweise ein Schütteln, wie es bei Smartphone bekannt geworden ist, eine bestimmte Funktion auszulösen, wie beispielsweise einen Programmwechsel.

Es versteht sich, dass bevorzugt der aktuelle Zustand des erfindungsgemäßen Lichthärtgeräts über einen hierfür geeignete Anzeige, wie eine Flüssigkristallanzeige, signalisiert wird.

Bei einer unabsichtlichen Verlagerung, wie beispielsweise einer allzu starken Winkelabweichung währen des Polymerisierens, oder auch beispielsweise beim Fallenlassen des Handgeräts, lässt sich ein Alarm auslösen und/oder das betreffende Ereignis protokollieren, was auch als Nachweis für Garantieansprüche ausgenutzt werden kann. Die Ortserfassung kann in an sich bekannter Weise über Triangulation gegenüber mindestens drei vorgegebenen Referenzquellen realisiert sein. Zu den in Frage kommenden Medien gehören elektromagnetische, aber auch Ultraschall- oder Infrarotquellen. Auch ist es möglich, über eine Kamera eine Bilderkennung vorzunehmen und hierdurch die Lageänderung zu erfassen. Bevorzugt erfolgt die Erkennung der Verlagerung oder Bewegung relativ, also bevorzugt bezogen auf einen Referenzpunkt, eine Referenzposition oder eine Referenzwinkellage.

Es ist auch möglich, bei einer geringen Abweichung von der erwünschten Position und/oder Winkelausrichtung die Lichtleistung gegebenenfalls kurzzeitig zu erhöhen und/oder erfindungsgemäß die Belichtungszeit zu verlängern, um dennoch eine ordnungsgemäße Polymerisation zu gewährleisten und die erforderliche Gesamtlichtdosis zu erreichen. Durch die Erfassung der Positionsabweichung oberhalb eines ersten Schwellenwerts wird bei dieser Lösung mindestens eine Kompensationsmaßnahme eingeleitet, während bei Erfassung einer Abweichung oberhalb eines zweiten oder weiteren Schwellenwerts (auch mehr als zwei) eine Signalisierung vorgenommen wird, eine Abspeicherung und/oder eine Ausschaltung des Lichthärtgeräts.

Zu den Möglichkeiten, die Fehlstellung zu kompensieren, zählt auch die Nachführung des Lichtkegels bzw. die Änderung der Fokusierung, was die Verlagerung entsprechender Linsen und/oder Reflektoren bedingt.

Es ist auch möglich, eine Minikamera auf die Bearbeitungsoberfläche zu richten und die Nachführung per Bilderkennung dann automatisch erfolgen zu lassen, wenn eine Verlagerung festgestellt wird. In diesem Fall dient insofern die Minikamera als Bewegungssensor bzw. Ortsensor.

Weitere Vorteile, Einzelheiten und Merkmale ergeben sich aus der nachfolgenden Beschreibung mehrerer Ausführungsbeispiele anhand der Zeichnung.

Es zeigen:
- Fig. 1: Eine schematische Ansicht eines erfindungsgemäßen Lichthärtgeräts, unter Einbeziehung von zwei Raumkoordinaten;
- Fig. 2: das Ausführungsbeispiel gemäß Fig. 1 in eine Queransicht und dementsprechend unter Darstellung von zwei anderen Raumkoordinaten;
- Fig. 3: eine modifizierte Ausführungsform des erfindungsgemäßen Lichthärtgeräts, das einen Rotationssensor aufweist;
- Fig. 4: eine Darstellung des Lichthärtgeräts gemäß Fig. 3 in einer anderen Ansicht;
- Fig. 5a und 5b: zwei Darstellungen der Handhabung eines Teils des erfindungsgemäßen Lichthärtgeräts, wobei der Abstand und die Relativposition zu einem zu behandelnden Zahn erfasst wird;
- Fig. 6: eine Darstellung der Verlängerung der Belichtungszeit bei Erfassung einer Winkelabweichung;
- Fig. 7: eine Darstellung einer weiteren Ausführungsform eines erfindungsgemäßen Lichthärtgeräts bei welchen ein Magnetfeld erfasst wird;
- Fig. 8: eine weitere Ausführungsform eines erfindungsgemäßen Lichthärtgeräts mit Ultraschall- oder Laserentfernungsmesser;
- Fig. 9: eine weitere Ausführungsform eines erfindungsgemäßen Lichthärtgeräts; und
- Fig. 10: eine weitere Ausführungsform eines erfindungsgemäßen Lichthärtgeräts.

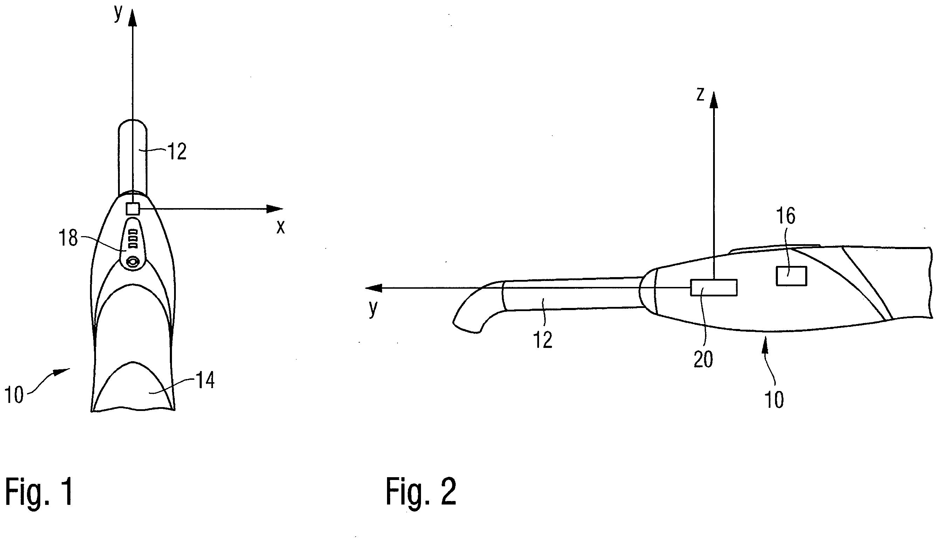

Das in Fig. 1 dargestellte Lichthärtgerät 10 weist in an sich bekannter Weise einen abgekröpften Lichtleiter 12 und ein Gehäuse 14 auf. Das Gehäuse 14 nimmt eine Lichtquelle auf, die das emittierte Licht dem Lichtleiter 12 zuleitet, und auch eine Energiequelle, in dem dargestellten Ausführungsbeispiel in Form eines Akkumulators. Eine in Fig. 2 schematisch dargestellte Steuereinrichtung 16 steuert den Polymerisationszyklus des Lichthärtgeräts 10. Zusätzlich ist ein Schalter 18 an dem Gehäuse 14 vorgesehen, in dem Ausführungsbeispiel an der Oberseite des im wesentlichen stiftförmigen und/oder pistolenförmigen Lichthärtgeräts 10.

Erfindungsgemäß weist das Lichthärtgerät 10 ferner einen Sensor 20 auf, der im dargestellten Ausführungsbeispiel als translatorischer Bewegungssensor ausgebildet ist. Der Sensor 20 gibt daher Bewegungsdaten des Lichthärtgeräts 10, die er erfasst, an die Steuereinrichtung 16 weiter; diese bewirkt die basierend auf der Bewegung erforderliche Aktion des Lichthärtgeräts 10.

In diesem Ausführungsbeispiel ist der Bewegungssensor 20 als translatorischer Bewegungssensor ausgeführt. Er erfasst Bewegungen des Lichthärtgeräts in drei Raumrichtungen, von denen in Fig. 1 die Raumrichtungen x und y und in Fig. 2 die Raumrichtungen y und z dargestellt sind.

Als beispielhafte Anwendung ist es bei dieser Ausführungsform vorgesehen, dass das Lichthärtgerät 10, wenn es denn abgestellt ist bzw. in einer Ruheposition ist, eine Bewegung in beliebiger Art erfasst. Wenn der Zahnarzt das Lichthärtgerät aufnimmt, erfolgt eine Bewegung, und basierend auf dem entsprechend erfassten Bewegungssignal des Sensors 20 überführt die Steuereinrichtung 16 das Lichthärtgerät 10 von einem Ruhezustand in den Bereitschaftszustand.

Während im Ruhezustand sämtliche energieintensiven Schaltkreise des Lichthärtgeräts abgeschaltet sind und lediglich der Bewegungssensor mit einem ganz geringen Strom versorgt wird, ist das Lichthärtgerät 10 im Bereitschaftszustand bereit, um unmittelbar, also verzögerungsfrei, einen Polymerisationszyklus zu starten. Die hierfür erforderlichen Stromkreise, also die eigentliche Steuerung des Lichthärtgeräts, werden hierzu mit Strom versorgt. In der Praxis ist der Strombedarf des Lichthärtgeräts im Ruhezustand ganz gering und beträgt beispielsweise 1µA oder sogar nur 100nA, während im Bereitschaftszustand ein Stromverbrauch von beispielsweise 20mA entsteht.

Es ist auch möglich, im Bereitschaftszustand einen Kondensator hoher Kapazität wie beispielsweise 10F, aufzuladen und hierfür beispielsweise einen Strom von einem 1A mit Start des Bereitschaftszustands diesem Kondensator zuzuleiten.

Der Stromverbrauch während des Bereitschaftszustands kann bei dieser Ausgestaltung dementsprechend in der Größenordnung des Stromverbrauchs des Polymerisationszyklus sein.

Erfindungsgemäß ist es bei dieser Ausführungsform daher besonders günstig, dass ein Ruhezustand möglich ist, in welchem der Stormverbrauch drastisch, also um mehrere Zehnerpotenzen, reduziert ist und daher die Laufzeit des verwendeten Akkumulators entsprechend erhöht wird, ohne dass sich Verzögerungen beim Startprogramm des Mikroprozessors für den Polymerisationszyklus ergeben - der Mikroprozessor ist natürlich im Ruhezustand abgeschaltet.

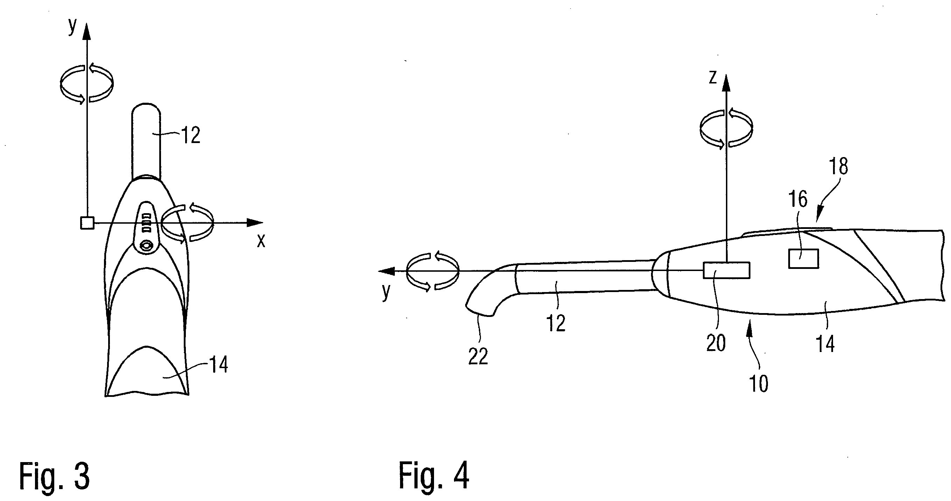

Aus Fig. 3 ist ein demgegenüber modifiziertes Lichthärtgerät ersichtlich. Dieses wie auch die weiteren sind für entsprechende Teile mit entsprechenden oder gleichen Bezugszeichen versehen, so dass die Bezugszeichen nicht erneut erläutert werden müssen.

Bei dem Mikroprozessor gemäß den Figuren 3 und 4 ist der Sensor 20 ebenfalls als Bewegungssensor ausgebildet, jedoch als Rotationssensor. Dieser hat eine demgegenüber anderen Funktion, die mit der Polymerisation zusammenhängt.

Beim Polymerisieren muss darauf geachtet werden, dass die Spitze 22 des Lichtleiters 12 unmittelbar und nach Möglichkeit in nahezu direktem Kontakt mit der zu polymerisierenden Dentalrestauration ist. Der Zahnarzt fasst das Lichthärtgerät 10 typischerweise am Gehäuse 14. Der Schalter 18 ist mit geeigneten Anzeigen wie LED-Anzeigen bestückt, um den Betriebszustand zu signalisieren, so dass dieser Bereich beim Greifen häufig freigelassen wird.

Wenn nun während der Polymerisation der Zahnarzt beispielsweise abgelenkt wird oder unabsichtlich eine unwillkürliche Bewegung der Hand ausführt, entfernt sich die Spitze 22 von dem Zielgebiet, also der Dentalrestauration. Aufgrund der Länge des Lichthärtgeräts 10 führt bereits eine ganz geringe Rotation um die Achse Z gemäß Fig. 4 zu einer starken Verlagerung der Spitze 22.

Erfindungsgemäß wirkt einer hierdurch indizierten Fehlpolymerisation der erfindungsgemäßse Sensor 20 in der Ausgestaltung als Rotationssensor entgegen, denn basierend auf dessen Ausgangssignal während des Polymerisationszyklus erzeugt die Steuervorrichtung 16 eine geeignete Signalisierung.

Es ist auch möglich, die Korrekturbewegungsrichtung anzuzeigen; wenn die Spitze 22 beispielsweise versehentlich nach links geschwenkt wird, wird im Bereich der Anzeige des Schalters 18 ein Signal eingeblendet "nach rechts drehen".

Dies kann durch entsprechende Piktogramme, eine beliebige andere Visualisierung oder beispielsweise auch durch Sprachausgabe realisiert werden.

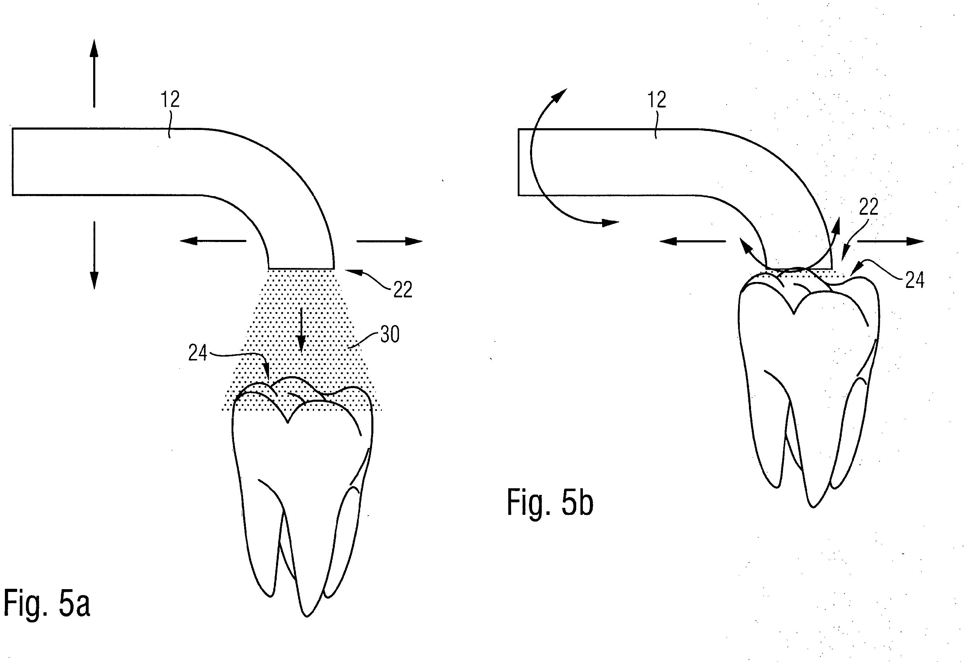

Aus Fig. 5a und 5b ist die Polymerisationssituation während des Polymerisationszyklus ersichtlich. In der Darstellung gemäß Fig. 5b ist die Spitze 22 des Lichtleiters 12 der Dentalrestauration 24 undmittelbar benachbart und die Polymerisation erfolgt mit maximaler Beleuchtungsstärke, also entsprechend rasch.

Demgegenüber ist in der Darstellung gemäß Fig. 5a die Spitze 22 des Lichtleiters 12 deutlich stärker von der Dentalrestauration 24 beabstandet. Aufgrund der Strahlaufweitung 30 ist die Belichtungsintensität der Dentalrestauration 24 geringer, und besteht nun die Möglichkeit, entweder ein Lagekorrektursignal abzugeben, oder aber erfindungsgemäß die Belichtungszeit automatisch zu verlängern.

Er versteht sich, dass auch die Kombination beider Möglichkeiten besteht, also die Belichtungszeit so lange zu verlängern, bis der Zahnarzt die Fehlpositionierung merkt und dann die Spitze 22 wieder in die Position gemäß Fig. 5b bringt.

Es versteht sich, dass auch bei einer seitlichen Verlagerung der Spitze 22 Fehlhärtungen entstehen können. Je nach Abmessung der Dentalrestauration 24 im Verhältnis zum Emissionsdurchmesser der Spitze 22 sind diese sogar kritischer als eine Abstandsveränderung, was erfindungsgemäß mit dem Lagesensor 20 kompensiert werden soll.

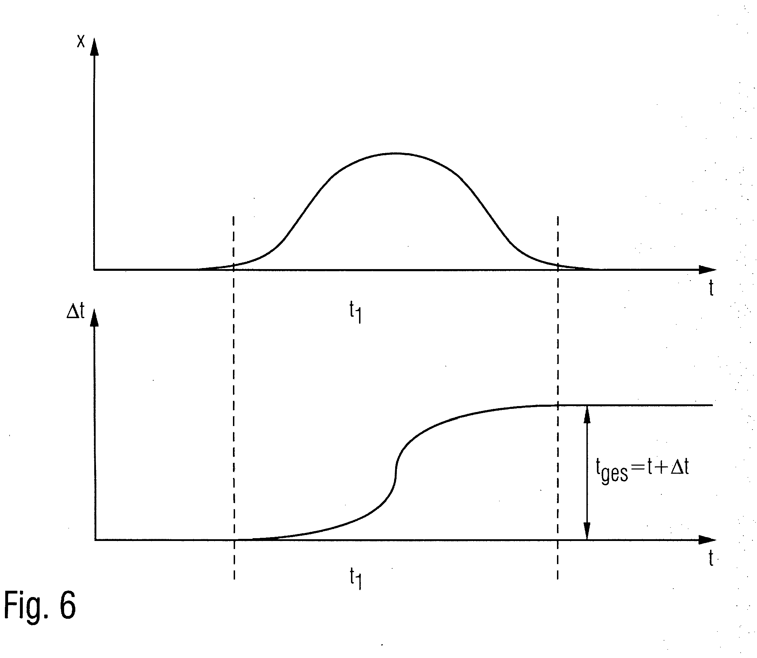

Aus Fig. 6 ist ersichtlich, in welcher Weise die Verlängerung der Belichtungszeit rechnerisch sichergestellt wird. Die obere Kurve aus Figur 6 zeigt eine versehentliche Verlagerung, beispielsweise in x-Richtung. Wenn die Verlagerung einen gewissen Schwellenwert übersteigt, beispielsweise zum Zeitpunkt t₁, wird ein Fehlersignal dem Zahnarzt übermittelt. Nach dessen Reaktionszeit sollte das Lichthärtgerät wieder an die zutreffende Position gebracht werden.

Ohne erfindungsgemäßen Sensor würde die wirksame Polymerisationszeit jedoch zu kurz sein, und daher wird erfindungsgemäß die Polymerisationszeit um den Wert ΔT, entsprechend der Zeit, in der die Verlagerung in die Fehlpostion besteht, verlängert.

Zwar verlängert sich erfindungsgemäß dadurch die Polymerisationszeit auf t_{ges}. Dies ist jedoch weniger bedeutsam als eine Fehlpolymerisation, die bekanntlich auch gravierende Folgen haben kann.

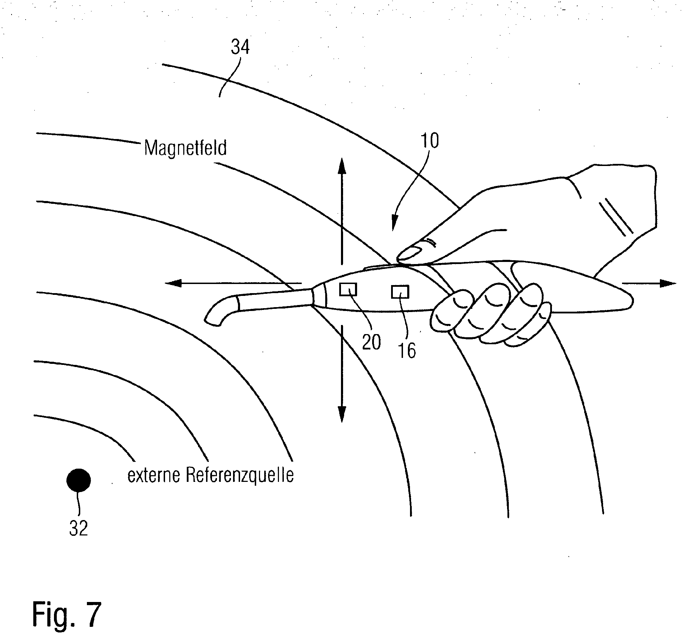

Aus Fig. 7 ist eine weitere Möglichkeit der Positionserfassung des erfindungsgemäßen Lichthärtgeräts dargestellt.

Es versteht sich, dass das Lichthärtgerät jedenfalls als Handgerät ausgebildet ist und entweder eine externe oder eine interne Stromquelle aufweist. In dieser Ausführungsform, die in Fig. 7 schematisch dargestellt ist, ist eine externe Referenzquelle 32 realisiert, die ein Magnetfeld erzeugt. Bei dieser Lösung lässt sich der Abstand und die Abstände zur externen Referenzquelle feststellen und damit mindestens eine Positionsbestimmung vornehmen.

In der Praxis sind mindestens 1, bevorzugt 3 externe Referenzquellen 32, die je Magnetfelder 34 erzeugen, bei dieser Ausführungsform realisiert, und in an sich bekannter Weise lässt sich die exakte Position durch Triangulation feststellen.

Der Sensor 20 ist in dem dargestellten Ausführungsbeispiel als Magnetfeldsensor ausgebildet.

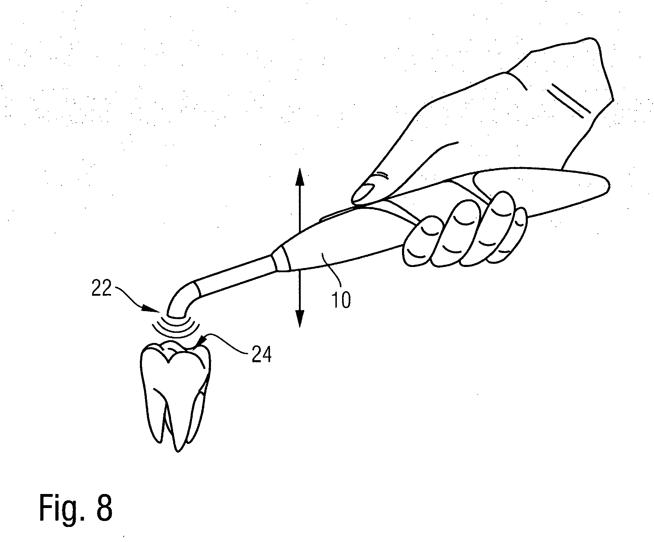

Bei der Ausführungsform gemäß Fig. 8 ist es vorgesehen, einen Abstandsensor an der Spitze 22 des Lichthärtgeräts 10 auszubilden. Der Abstandsensor kann beispielsweise mittels Ultraschall oder als Laserentfernungsmesser realisiert sein. Hierdurch lässt sich unmittelbar die Relativposition des Lichthärtgeräts 10 zur Behandlungsoberfläche, also der Dentalrestauration, feststellen.

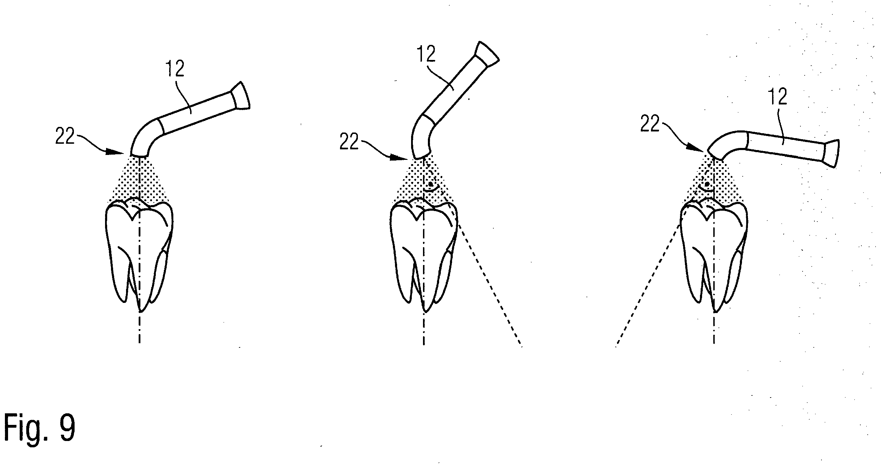

Aus Fig. 9 ist ersichtlich, dass über einen Rotationssensor auch die Winkelausrichtung der Emissionsachse erfasst und gegebenenfalls korrigiert werden kann.

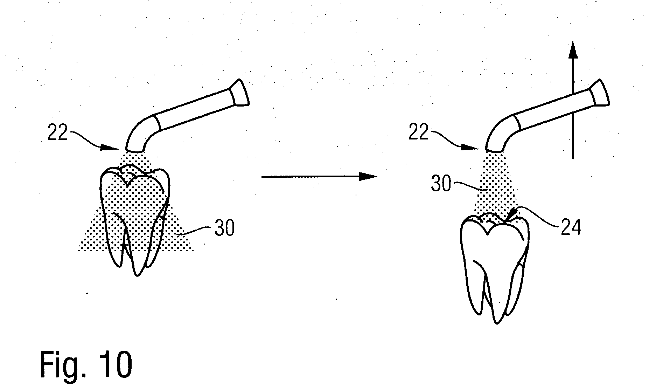

Fig. 10 zeigt schematisch, in welcher Weise die Lichtaufweitung 30 in Kombination mit dem Abstand der Spitze 22 von der Dentalrestauration 24 Auswirkungen auf die Bestrahlung hat. Bei der links dargestellten vergleichsweise großen Strahlaufweitung 30 reicht schon ein geringer Abstand aus, um auch eine großflächige Dentalrestauration zu beaufschlagen. Demgegenüber ist bei der rechts dargestellten Strahlaufweitung 30 ein größerer Abstand vonnöten, um die gesamte großflächige Dentalrestauration zu beaufschlagen.

Insofern besteht stets ein optimaler Abstand zwischen der Spitze 22 und der Dentalrestauration 24, und der erfindungsgemäße Bewegungssensor kann diesen nicht nur feststellen, sondern er kann auch signalisiert bzw. korrigiert werden.

In der Ausführungsform gemäß Fig. 10 ist es vielmehr vorgesehen, auch die Fokussierung der Strahlaufweitung 30 an den Abstand anzupassen, was in an sich bekannter Weise mit optischen Mitteln erfolgen kann.

## Patentansprüche

1. Lichthärtgerät(10), mit einer Lichtquelle und einem Lichtabgabeelement wie einem Lichtleiter (12), dessen Lichtauslassende dazu ausgebildet ist, auf ein zu polymerisierendes Material gerichtet zu werden, mit einer oder mehreren Steuereinrichtungen (16), die zum Einschalten der Lichtquelle während eines Poylmerisationszyklus vorgesehen ist/sind, und mit einem oder mehreren Sensoren (20) oder Sensorenkombinationen, der/die an die mindestens eine Steuereinrichtung (16) angeschlossen ist/sind,
wobei ein Sensor (20) als als Bewegungssensor (20) ausgebildet ist, der geeignet ist, eine Bewegung des als Handgerät ausgebildeten Lichthärtgeräts (10) zu erfassen und Signale, die die Bewegung wiedergeben, der Steuereinrichtungen (16) zuzuleiten, **dadurch gekennzeichnet, dass** die mindestens eine Steuereinrichtung (16) dazu ausgebildet ist, über den Bewegungssensor (20) bei Beginn des Poymerisationszyklus, eine optimale Lage des Lichtabgabeelements relativ zu dem zu polymerisierenden Material festzulegen und den Polymerisationszyklus zu verlängern, wenn basierend auf den Signalen des Bewegungssensors (20) eine Abweichung von dieser Lage während des Polymerisationszyklus entsteht, und zwar proportional dem Grad der Abweichung und insbesondere der Zeitdauer der Abweichung.

2. Lichthärtgerät nach Anspruch 1, **dadurch gekennzeichnet, dass** die Steuereinrichtung die Lage des Lichtabgabeelements relativ zu dem zu polymerisierenden Material erfasst.

3. Lichthärtgerät nach einem der Ansprüche 1 oder 2, **dadurch gekennzeichnet, dass** die mindestens eine Steuereinrichtung (16) an einen Lichtsensor und/oder eine Kamera angeschlossen ist, der/die mit dem als Handgerät ausgebildeten Lichthärtgerät (10) verbunden ist/sind und auf das zu polymerisierende Material oder die Behandlungsfläche gerichtet ist/sind, und dass basierend auf den Ausgangssignalen des Lichtsensors und/oder der Kamera die optimale Lage des Lichthärtgeräts (10) für den Polymerisationszyklus festlegbar ist.

4. Lichthärtgerät nach einem der vorhergehenden Ansprüche, **dadurch gekennzeichnet, dass** mindestens ein Bewegungssensor (20) als Relativbewegungssensor ausgebildet ist, der eine Relativbewegung bezogen auf die Position und/oder Neigung des Handgeräts bei Belichtungsstart, also bei Beginn des Polymerisationszyklus in alle drei Dimensionen erfasst.

5. Lichthärtgerät nach einem der vorhergehenden Ansprüche, **dadurch gekennzeichnet, dass** mindestens ein Sensor als Gyrosensor oder Winkelsensor ausgebildet ist.

6. Lichthärtgerät nach einem der vorhergehenden Ansprüche, **dadurch gekennzeichnet, dass** das Lichthärtgerät (10) auch eine Basisstation umfasst, die ein Referenzfeld erzeugt, und dass ein Sensor (20), insbesondere ein Magnetssensor, eine Änderung des Orts und/oder der Bewegung und/oder der Beschleunigung in dem Referenzfeld, insbesondere in mindestens zwei Dimensionen, erfasst.

7. Lichthärtgerät nach einem der vorhergehenden Ansprüche, **dadurch gekennzeichnet, dass** mindestens ein Sensor (20) des Handgeräts eine Lageerfassung über Triangulation von GPS-Satelliten, über Funksender, über elektromagnetische Felder und/oder über Ultraschall und/oder Infrarot aufweist.

8. Lichthärtgerät nach einem der vorhergehenden Ansprüche, **dadurch gekennzeichnet, dass** das Handgerät einen Ortsensor zur Erfassung des Abstands zu Behandlungsoberfläche, also zu dem zu polymerisierenden Material, aufweist, der den Abstand über die sich verändernde Helligkeit eines vorgegebenen Referenzlichtstrahls erfasst.

9. Lichthärtgerät nach einem der vorhergehenden Ansprüche, **dadurch gekennzeichnet, dass** die Steuereinrichtung (16) die Belichtungszeit, also die Länge des Polymerisationszyklus, in Abhängigkeit von dem Signal des oder der Sensoren (20) anpasst und steuert und bei einer Fehlposition und/oder einer Bewegung des Handstücks die Belichtungszeit verlängert oder bei der Erreichung eines Abbruchkriteriums/Schwellenwerts das Lichtgerät abschaltet.

10. Lichthärtgerät nach einem der vorhergehenden Ansprüche, **dadurch gekennzeichnet, dass** die Steuereinrichtung (16) bei einer Fehlpositionierung/Bewegung ab einem oder mehreren vorgegebenen Schwellenwert die Bestrahlungsstärke, insbesondere kurzzeitig, erhöht oder bei Erreichung eines Abbruchkriteriums/Schwellenwerts das Lichtgerät abschaltet.

11. Lichthärtgerät nach einem der vorhergehenden Ansprüche, **dadurch gekennzeichnet, dass** das die Steuereinrichtung (16) eine Fehlposition des Lichthärtgeräts (10) während des Polymerisationszyklus, insbesondere basierend auf den erfassten Signalen des oder der Sensoren (20), durch eine geeignete Rückmeldung dem Benutzer signalisiert, insbesondere durch Vibration und/oder durch eine akustische/visuelle Signalisierung, insbesondere durch eine leuchtende LED im Gehäuse (14), und/oder durch Abschaltung des Lichthärtgeräts (10).

12. Lichthärtgerät nach Anspruch 11, **dadurch gekennzeichnet, dass** mit der Steuervorrichtung bei einer von dem oder den Sensoren (20) erfassten Fehlposition, die von der Startposition ab einem vorgegebenen Schwellenwert unterschiedlich ist, der von dem Lichtauslassende emittierte Lichtkegel durch eine Ablenkvorrichtung wie einen Reflektor oder eine Linse nachführbar ist.

13. Lichthärtgerät nach einem der vorhergehenden Ansprüche, **dadurch gekennzeichnet, dass** mindestens ein Bewegungssensor (20) als Beschleunigungssensor ausgebildet ist und bei einer Beschleunigung, die oberhalb eines vorgegebenen Wertes ist, die Steuereinrichtung (16) diese Beschleunigung auswertet und abspeichert, um eine übermäßige Erschütterung des Lichthärtgeräts (10) nachzuweisen.

14. Lichthärtgerät nach einem der vorhergehenden Ansprüche, **dadurch gekennzeichnet, dass** die Steuereinrichtung eine Kombination aus Beschleunigungssensor, Gyrosensor und Magnetfeldsensor und deren Einzelsignal zu einem Gesamtsignal verarbeitet und das Rauschen bzw. die Fehlinformation kompensiert und ein konstantes Signal erzeugbar ist.

15. Lichthärtgerät nach einem der vorhergehenden Ansprüche, **dadurch gekennzeichnet, dass** die Steuereinrichtung die Fokussierung des Lichtaustritts in Abhängigkeit von der Bewegung/Fehlposition des Handstücks reguliert bzw. erhöht und damit lokal die Bestrahlungsstärke erhöht.

## Claims

1. A light curing device (10), comprising a light source and a light emitting element such as a light guide (12), the light emitting end of which light guide is adapted to be directed onto a material to be polymerized, one or more control device (16) being provided for switching on the light source during a polymerization cycle, and one or more sensors (20) or sensor combinations which is/are connected to the at least one control device (16), wherein a sensor (20) is designed as a movement sensor (20) which is suitable for detecting a movement of the light-curing device (10) designed as a hand-held device, and guiding signals, which represent the movement, to the control device (16), **characterized in that** the at least one control device (16) is designed to determine, via the movement sensor (20) at the beginning of the polymerization cycle, an optimum position of the light-emitting element relative to the material to be polymerized and to extend the polymerization cycle if, based on the signals of the movement sensor (20), a deviation from this position occurs during the polymerization cycle, proportional to the degree of the deviation and in particular the duration of the deviation.

2. The light curing device according to claim 1, **characterized in that** the control device detect the position of the light emitting element in relation to the material to be polymerized.

3. The light curing device according to one of claims 1 or 2, **characterized in that** the at least one control device (16) is connected to a light sensor and/or a camera which is/are connected to the light curing device (10) designed as a hand-held device and is/are directed towards the material to be polymerized or the treatment surface, and **in that** the optimum position of the light curing device (10) for the polymerization cycle is allowed to be determined based on the output signals of the light sensor and/or the camera.

4. The light curing device according to one of the preceding claims, **characterized in that** at least one movement sensor (20) is designed as a relative movement sensor which detects a relative movement in all three dimensions in relation to the position and/or inclination of the hand-held device at the start of exposure, i.e. at the start of the polymerization cycle.

5. The light curing device according to one of the preceding claims, **characterized in that** at least one sensor is configured as a gyro sensor or angle sensor.

6. The light curing device according to one of the preceding claims, **characterized in that** the light curing device (10) also comprises a base station which generates a reference field, and that a sensor (20), in particular a magnetic sensor, detects a change of location and/or movement and/or acceleration in the reference field, in particular in at least two dimensions.

7. The light curing device according to one of the preceding claims, **characterized in that** at least one sensor (20) of the hand-held device comprises a position detection mechanism via triangulation of GPS satellites, via radio transmitters, via electromagnetic fields and/or via ultrasound and/or infrared.

8. The light curing device according to one of the preceding claims, **characterized in that** the hand-held device comprises a location sensor for detecting the distance to the treatment surface, i.e. to the material to be polymerized, which sensor detects the distance via the changing brightness of a predetermined reference light beam.

9. The light curing device according to one of the preceding claims, **characterized in that** the control device (16) adapts and controls the exposure time, i.e. the length of the polymerization cycle, depending on the signal of the sensor or sensors (20) and, in the event of malposition and/or displacement of the handpiece, extends the exposure time or switches off the light device when a termination criterion/threshold value is reached.

10. The light curing device according to one of the preceding claims, **characterized in that** the control device (16) increases irradiation intensity, in particular for a short time, in the event of malpositioning/movement above one or more predetermined threshold values or switches off the light device when reaching a termination criterion/threshold value.

11. The light curing device according to one of the preceding claims, **characterized in that** the control device (16) signals malpositioning of the light curing device (10) during the polymerization cycle, in particular based on the signals detected by the sensor(s) (20), to the user by means of a suitable feedback, in particular by vibration and/or by means of acoustic/visual signaling, in particular by a luminous LED in the housing (14), and/or by switching off the light curing device (10).

12. The light curing device according to claim 11, **characterized in that** using the control device in the event of a malposition detected by the sensor or sensors (20), which is different from the starting position from a predetermined threshold value, the light cone emitted by the light-emitting end can be tracked by a deflecting device such as a reflector or a lens.

13. The light curing device according to one of the preceding claims, **characterized in that** at least one movement sensor (20) is configured as an acceleration sensor and, in the event of acceleration being above a predetermined value, the control device (16) evaluates and stores said acceleration in order to detect excessive vibration of the light curing device (10).

14. The light curing device according to one of the preceding claims, **characterized in that** the control device processes a combination of acceleration sensor, gyro sensor and magnetic field sensor and their individual signals to form a total signal and the noise or malinformation is compensated and a constant signal may be generated.

15. The light curing device according to one of the preceding claims, **characterized in that** the control device regulates or increases the focusing of the light output as a function of the movement/malposition of the handpiece, thus locally increasing the irradiance.

## Revendications

1. Dispositif de photopolymérisation (10) avec une source de lumière et un élément émetteur de lumière tel qu'un guide de lumière (12), dont l'extrémité émettrice de lumière est adaptée pour être dirigée vers un matériau à polymériser, un ou plusieurs moyens de commande (16) qui est/sont prévus pour allumer la source de lumière pendant un cycle de polymérisation, et avec un ou plusieurs capteurs (20) ou combinaisons de capteurs qui est/sont relié(s) audit au moins un dispositif de commande (16), où un capteur (20) est conçu comme un détecteur de mouvement (20) qui est adapté pour détecter un mouvement du dispositif de photopolymérisation (10) conçu comme un dispositif portatif, et diriger des signaux, qui représentent le mouvement, vers les dispositifs de commande (16), **caractérisé en ce que** ledit au moins un dispositif de commande (16) est conçu pour déterminer, par l'intermédiaire du détecteur de mouvement (20) au début du cycle de polymérisation, une position optimale de l'élément émetteur de lumière par rapport au matériau à polymériser et pour prolonger le cycle de polymérisation si, sur la base des signaux du détecteur de mouvement (20), un écart par rapport à cette position se produit pendant le cycle de polymérisation, proportionnellement au degré de l'écart et en particulier à la durée de l'écart.

2. Dispositif de photopolymérisation selon la revendication 1, **caractérisé en ce que** le moyen de commande détecte la position de l'élément émetteur de lumière par rapport au matériau à polymériser.

3. Dispositif de photopolymérisation selon l'une des revendications 1 ou 2, **caractérisé en ce que** ledit au moins un dispositif de commande (16) est relié à un capteur de lumière et/ou à une caméra qui est/sont relié(s) au dispositif de photopolymérisation (10) conçu comme un dispositif portatif et est/sont dirigé(s) vers le matériau à polymériser ou la surface de traitement, et **en ce que** la position optimale du dispositif de photopolymérisation (10) pour le cycle de polymérisation peut être déterminée sur la base des signaux de sortie du capteur de lumière et/ou de la caméra.

4. Dispositif de photopolymérisation selon l'une des revendications précédentes, **caractérisé en ce qu'**au moins un détecteur de mouvement (20) est conçu comme un détecteur de mouvement relatif qui détecte un mouvement relatif dans les trois dimensions par rapport à la position et/ou à l'inclinaison du dispositif portatif au début du exposition, c'est-à-dire au début du cycle de polymérisation.

5. Dispositif de photopolymérisation selon l'une des revendications précédentes, **caractérisé en ce qu'**au moins un capteur est conçu comme un capteur gyroscopique ou un capteur d'angle.

6. Dispositif de photopolymérisation selon l'une des revendications précédentes, **caractérisé en ce que** le dispositif de photopolymérisation (10) comprend également une station de base, qui génère un champ de référence, et **en ce qu'**un capteur (20), en particulier un capteur magnétique, détecte un changement de position et/ou de mouvement et/ou d'accélération dans le champ de référence, en particulier dans au moins deux dimensions.

7. Dispositif de photopolymérisation selon l'une des revendications précédentes, **caractérisé en ce qu'**au moins un capteur (20) du dispositif portatif présente une détection de position par triangulation de satellites GPS, par des émetteurs radio, par des champs électromagnétiques et/ou par des ultrasons et/ou des infrarouges.

8. Dispositif de polymérisation par la lumière selon l'une des revendications précédentes, **caractérisé en ce que** le dispositif portatif présente un capteur de localisation pour détecter la distance par rapport à la surface de traitement, c'est-à-dire par rapport au matériau à polymériser, qui détecte la distance par l'intermédiaire de la luminosité changeante d'un faisceau lumineux de référence prédéterminé.

9. Dispositif de photopolymérisation selon l'une des revendications précédentes, **caractérisé en ce que** le dispositif de commande (16) adapte et commande le temps d'exposition, c'est-à-dire la longueur du cycle de polymérisation, en fonction du signal du ou des capteurs (20) et, en cas de position et/ou de mouvement défectueux de la pièce à main, prolonge le temps d'exposition ou éteint le dispositif de photopolymérisation lorsqu'un critère d'interruption/valeur seuil est atteint.

10. Dispositif de photopolymérisation selon l'une des revendications précédentes, **caractérisé en ce que** le dispositif de commande (16) augmente l'intensité de l'irradiation, en particulier pour une courte durée, en cas de positionnement incorrect ou de mouvement au-dessus d'une ou de plusieurs valeurs seuils prédéterminées ou éteint le dispositif de photopolymérisation lorsqu'un critère d'interruption/valeur seuil est atteint.

11. Dispositif de photopolymérisation selon l'une des revendications précédentes, **caractérisé en ce que** le dispositif de commande (16) signale à l'utilisateur un mauvais positionnement du dispositif de photopolymérisation (10) pendant le cycle de polymérisation, en particulier sur la base des signaux détectés du ou des capteurs (20), au moyen d'une rétroaction appropriée, en particulier par vibration et/ou au moyen d'une signalisation acoustique/visuelle, en particulier par une LED lumineuse dans le boîtier (14), et/ou en éteignant le dispositif de photopolymérisation (10).

12. Dispositif de photopolymérisation selon la revendication 11, **caractérisé en ce qu'**avec le dispositif de commande, en cas de position défectueuse détectée par le ou les capteurs (20), qui est différente de la position de départ à partir d'une valeur seuil prédéterminée, le cône de lumière émis par l'extrémité émettrice de lumière peut être suivi par un dispositif de déviation tel qu'un réflecteur ou une lentille.

13. Dispositif de photopolymérisation selon l'une des revendications précédentes, **caractérisé en ce qu'**au moins un détecteur de mouvement (20) est réalisé sous la forme d'un capteur d'accélération et **en ce que**, pour une accélération qui est supérieure à une valeur prédéterminée, le dispositif de commande (16) évalue et mémorise cette accélération afin de détecter une vibration excessive du dispositif de photopolymérisation (10).

14. Dispositif de photopolymérisation selon l'une des revendications précédentes, **caractérisé en ce que** le dispositif de commande traite une combinaison de capteur d'accélération, de capteur gyroscopique et de capteur de champ magnétique et leur signal individuel pour former un signal total et le bruit ou la désinformation est compensé et un signal constant peut être généré.

15. Dispositif de photopolymérisation selon l'une des revendications précédentes, **caractérisé en ce que** le dispositif de commande régule ou augmente la focalisation de l'émission de lumière en fonction du déplacement/mise en place de la pièce à main et augmente ainsi localement l'intensité de l'irradiation.
